# EUROPÄISCHE PATENTSCHRIFT

(11) **EP 1 727 574 B1**
(45) Veröffentlichungstag und Bekanntmachung des Hinweises auf die Patenterteilung: **04.06.2008**
(21) Anmeldenummer: 05715888.3
(22) Anmeldetag: 09.03.2005
(51) Int. Cl.: A61M 1/34, A61M 1/36

(54) **VORRICHTUNG ZUM WECHSELN UND/ODER ANDOCKEN VON FUNKTIONSMODULEN**
DEVICE FOR EXCHANGING AND/OR DOCKING FUNCTIONAL MODULES
DISPOSITIF CONCU POUR ECHANGER ET/OU INSERER DES MODULES FONCTIONNELS

(30) Priorität: 09.03.2004 DE 102004011461
(43) Veröffentlichungstag der Anmeldung: 06.12.2006
(73) Patentinhaber: PPA Technologies AG, 07745 Jena (DE)
(72) Erfinder: FERRARI, Markus, 07747 Jena (DE)
(74) Vertreter: Weidener, Jörg Michael
(86) Internationale Anmeldenummer: PCT/EP2005/002505
(87) Internationale Veröffentlichungsnummer: WO 2005/087290

(56) Entgegenhaltungen:
- US-A- 5 422 057
- US-B1- 6 241 945

## Beschreibung

Die vorliegende Erfindung betrifft eine Vorrichtung zum Wechseln und/oder Andocken von Funktionsmodulen für die extrakorporale Zirkulation von Körperflüssigkeiten, wobei jedes Funktionsmodul wenigstens eine von der Körperflüssigkeit durchströmbare Leitung mit einem Einlass und einem Auslass aufweist.

Bei dem Einsatz von Maschinen, welche mit Blut oder sterilen Flüssigkeiten (z. B. zur Verabreichung von Medikamenten) durchflossen sind, ist es bekannt, Leitungsverbindungen durch das Zusammenstecken der die Leitungen bildenden Schläuche oder durch Schraubadapter herzustellen. Dies ist insbesondere bei der Hämodialyse oder auch beim Betrieb von Herz-/Lungenmaschinen notwendig, wobei immer auf eine ausreichende Sterilität der Verbindungen geachtet werden muss. Entsprechend bergen diese bekannten Systeme der extrakorporalen Zirkulation während des Betriebes am Patienten die Gefahr eines Blutaustrittes in die Umgebung oder eines Keimeintrittes in das Perfusionssystem und damit in den Kreislauf des Patienten. Das Umstecken von einem Funktionsmodul des Perfusionssystems auf ein zusätzliches oder auf ein anderes Modul (z. B. beim Wechsel eines Dialysefilters bei der Hämodialyse oder des Oxygenators bei der Herz-/Lungenmaschine) muss in der Regel sehr rasch erfolgen, da ansonsten mit Schäden für den Patienten und mit einer konsekutiven Fehlfunktion des Perfusionssystems (z. B.

Thrombusbildung, Lufteintritt) zu rechnen ist. Bei der chronischen Hämodialyse hat es sich aus diesem Grunde etabliert, bei verstopftem Filter das gesamte Perfusionssystem einschließlich der Hähne, Adapter usw. zu wechseln. Dies bedeutet jedoch ein Dekonnektieren des Gerätes vom Patientenkreislauf mit entsprechender Infektionsgefahr, Blutverlust (das Blut in den Verbindungsschläuchen kann nie vollständig reinfundiert werden) und Zeitverlust bei der Therapie. Außerdem entstehen im Falle eines Wechsels des gesamten Sets, also der Verbindungsschläuche und der übrigen Komponenten des Kreislaufes, die eigentlich weiter genützt werden könnten, zusätzliche Kosten. Beim Betrieb einer Herz-/Lungenmaschine, welche im Falle eines Sistierens der Herzfunktion des Patienten ohne Unterbrechung laufen muss, stellt der Wechsel eines defekten Membranoxygenators ein vital bedrohliches Manöver dar.

Das Dokument US-B1-6 241 945 wird als nächstliegender Stand der Technik angesehen. Es zeigt eine Vorrichtung die alle Merkmale des Oberbegriffs des Anspruchs 1 aufweist.

Vor dem Hintergrund dieser Problematik liegt der vorliegenden Erfindung die Aufgabe zugrunde, Funktionsmodule eines extrakorporalen Kreislaufs möglichst schnell und ohne ein Lösen von Schlauchverbindungen und ohne entsprechende Neukonnektion wechseln zu können.

Diese Aufgabe wird bei einer Vorrichtung zum Wechseln und/oder Andocken von Funktionsmodulen der eingangs genannten Art erfindungsgemäß durch Mittel zur Verbindung der Leitung eines ersten Funktionsmoduls mit dem Einlass und dem Auslass eines zweiten Funktionsmoduls gelöst, wobei jene Mittel die Verbindung derart herstellen, dass das erste Funktionsmodul durch das zweite Funktionsmodul strömungstechnisch überbrückt und die Körperflüssigkeit durch das zweite Funktionsmodul hindurch umgeleitet wird.

Die Vorteile der erfindungsgemäßen Lösung liegen insbesondere darin, dass bei laufender extrakorporaler Zirkulation eine Umleitung des Körperflüssigkeitsstroms von einem alten (ersten) Funktionsmodul in ein neues (zweites) Funktionsmodul hergestellt wird. Somit kann der Ersatz von Funktionsmodulen in der extrakorporalen Zirkulation ohne das Umstecken von Schlauchverbindungen und ohne Unterbrechung des Kreislaufs erfolgen. Somit ist es ein großer Vorteil der erfindungsgemäßen Vorrichtung, dass ein Wechsel der Funktionsmodule in möglichst kurzer Zeit erfolgen kann, während das Umstecken der Schlauchverbindungen bei bekannten Vorrichtungen auch in den Händen eines geübten Kardiotechnikers zu einem Stillstand der Herz-/Lungenmaschine von ein bis zwei Minuten führen kann. Dieses wird mit der vorliegenden Erfindung sicher vermieden.

Vorteilhafte Weiterbildungen der Erfindung sind in den Unteransprüchen angegeben. Vorzugsweise enthalten die Verbindungsmittel je einen Ventilstutzen für den Einlass und den Auslass des zweiten Funktionsmoduls sowie je ein zugeordnetes Portal in der Leitung des ersten Funktionsmoduls, wobei die Ventilstutzen beim Konnektieren der Funktionsmodule derart in das jeweils zugeordnete Portal eingreifen, dass der Funktionskreislauf des ersten Funktionsmoduls überbrückt ist. Somit stellen die Ventilstutzen in Art von Steckverbindern die Verbindung zwischen dem alten (ersten) und dem neuen (zweiten) Funktionsmodul und damit unter sterilen Bedingungen eine Umleitung des Blutflusses her. Dabei erlaubt die Positionierung der Ansteckpunkte für die Ventilstutzen den mehrmaligen Ersatz eines verbrauchten oder defekten (ersten) Funktionsmoduls durch ein neues (zweites) Funktionsmodul. Das zweite Funktionsmodul und jedes weitere, dieses wiederum ersetzende Funktionsmodul fungieren somit als "Bypass" zum verbrauchten bzw. zu ersetzenden (ersten) Funktionsmodul.

Während die Ventilstutzen für den Einlass und den Auslass des zweiten Funktionsmoduls grundsätzlich als Verbrauchsmaterial einzustufende Einzelteile sein können, ist vorzugsweise vorgesehen, dass der Einlass und der Auslass des zweiten Funktionsmoduls baulich mit je einem Ventilstutzen versehen sind, die dann beim Zusammenstecken der Funktionsmodule mit den entsprechend ausgebildeten Portalen zur Herstellung einer flüssigkeitsdichten, von der Körperflüssigkeit durchströmbaren Verbindung zusammenwirken. Somit weist dann ein zweites oder jedes weitere Funktionsmodul bereits baulich die beiden Ventilstutzen auf, deren standardisierte Anordnung dafür Sorge trägt, dass die Ventilstutzen beim Aufsetzen des zweiten oder jedes weiteren Funktionsmoduls auf das erste Funktionsmodul ohne weiteres in die zugeordneten Portale des ersten Funktionsmoduls eingreifen und somit die Verbindung zwischen den Funktionsmodulen und damit die Überbrückung des Funktionskreislaufs des ersten bzw. jedes vorangehenden Funktionsmoduls rasch und problemlos gewährleistet ist.

Um gegebenenfalls auf das zweite Funktionsmodul ein weiteres Funktionsmodul aufstecken zu können, ist in vorteilhafter Weise vorgesehen, dass auch das zweite Funktionsmodul zum Anschluss eines weiteren Funktionsmoduls ebenfalls mit Portalen versehen ist. Dadurch kann der Ersatz von Funktionsmodulen durch die Konfiguration der Module in gleicher Weise mehrmalig erfolgen.

Um dafür Sorge zu tragen, dass jedes der Portale nach außen hin keimfrei verschlossen ist, sind zwei erfindungsgemäße Alternativen vorgesehen: Zum einen kann jedes Portal am nach außen weisenden Ende im ungebrauchten Zustand mittels einer durchstoßbaren Membran verschlossen sein, oder aber dies geschieht mittels eines Ventils. Beide Einrichtungen können darüber hinaus durch eine Schutzkappe, durch eine Folie oder durch eine andersartige Abdeckung keimfrei oder gar steril gehalten werden. Auch eine Kombination dieser drei beispielhaft genannten Mittel ist denkbar.

Vorzugsweise ist der Auslass des zweiten Funktionsmoduls bzw. jedes weiteren Funktionsmoduls mit einem Priming-Ventil zum Entlüften des zweiten Funktionsmoduls bzw. des weiteren Funktionsmoduls oder zum Befüllen des selben mit einem Fluid versehen. Dieses Priming-Ventil kann selbstverständlich auch zur Verabreichung von Medikamenten genutzt werden.

Als bauliche Realisierung dessen, dass beim Konnektieren der Funktionsmodule der Kreislauf der Körperflüssigkeit durch das zweite Funktionsmodul hindurch umgeleitet und gleichzeitig der Kreislauf durch das erste Funktionsmodul unterbrochen wird, sind erfindungsgemäß zwei alternative Lösungen vorgesehen: Zum einen kann jedes Portal eine winklige Abzweigung der zugehörigen Leitung enthalten, so dass jeder Ventilstutzen beim Eindringen in das ihm zugeordnete Portal den abzweigenden Abschnitt der Leitung verschließt und den Durchfluss der Körperflüssigkeit in das zweite Funktionsmodul bzw. in jedes weitere Funktionsmodul umleitet. Dies ist die einfachste und gleichzeitig verlässlichste bauliche Gestaltung, da durch den Kardiotechniker bzw. Arzt keine weitere Maßnahme zum Umleiten des Kreislaufs ergriffen werden muss und stellt darüber hinaus die schnellste Verbindungsherstellung dar. Zum anderen kann aber auch vorgesehen sein, dass jedes Portal einen Ventilmechanismus in Form eines Drei-Wege-Ventils anstelle der Abzweigung enthält, welches dann nach dem Aufsetzen des zweiten Funktionsmoduls auf dessen Kreislauf umgestellt wird.

Im Folgenden wird ein bevorzugtes Ausführungsbeispiel der Erfindung anhand einer Zeichnung näher erläutert.

Es zeigen:
- Fig. 1:: Eine schematische Darstellung zweier Funktionsmodule mit einem weiteren, gestrichelt dargestellten Funktionsmodul;
- Fig. 2:: Die schematische Darstellung eines ersten Ausführungsbeispiels eines Portals in drei Konnektions-Phasen; und
- Fig. 3:: Die schematische Darstellung eines zweiten Ausführungsbeispiels eines Portals in drei Konnektions-Phasen.

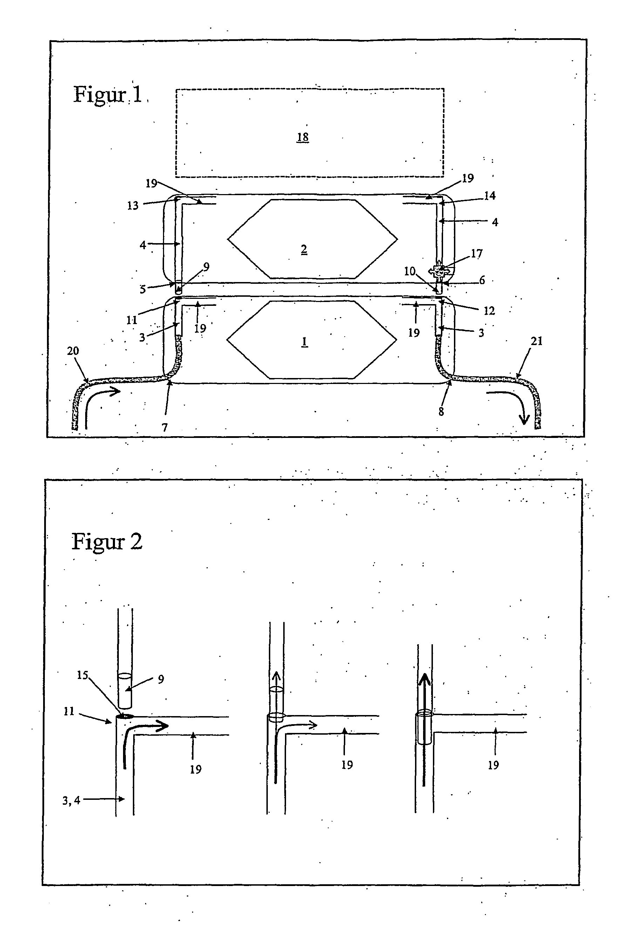

Figur 1 zeigt eine schematische Darstellung eines ersten Funktionsmoduls 1, eines zweiten Funktionsmoduls 2 und - gestrichelt dargestellt - eines weiteren Funktionsmoduls 18. Die Funktionsmodule 1, 2 und 18 sind von Körperflüssigkeiten wie beispielsweise Blut durchflossene Einheiten eines Perfusionssystems, wobei ein solches Funktionsmodul beispielsweise ein Dialysefilter für die Hämodialyse oder ein Oxigenator einer Herz-/Lungenmaschine sein kann. Dabei ist für die Zwecke der folgenden Beschreibung das Funktionsmodul 1 ein zu ersetzendes, verbrauchtes Funktionsmodul, bei dem beispielsweise der Filter verstopft ist, und das Funktionsmodul 2 ist ein neues, welches die Tätigkeit des Funktionsmoduls 1 übernehmen, dieses also ersetzen soll. Das gestrichelt dargestellte Funktionsmodul 18 ist lediglich ein weiteres Modul zur Darstellung der Möglichkeit, auch das Funktionsmodul durch ein weiteres Funktionsmodul, nämlich das Funktionsmodul 18, zu ersetzen.

Das Funktionsmodul 1 weist eine Leitung 3 für die extrakorporale Durchströmung mit einer Körperflüssigkeit auf, welche über eine Zuleitung 20 vom Patienten durch einen Einlass 7 in das Funktionsmodul 1 eintritt und über einen Auslass 8 und eine Ableitung 21 das Funktionsmodul 1 wieder verlässt und dem Patienten zugeführt wird. Die das Funktionsmodul 1 durchlaufende Leitung 3 weist an zwei von außen gut zugänglichen Stellen je ein Portal 11, 12 auf, welches einen Zugang zur Leitung 3 von außen ermöglicht. Dieser Zugang ist im unbenutzten Zustand des Portals 11, 12 durch eine durchstoßbare Membran 15 keimfrei verschlossen. Die Membran 15 ist gegebenenfalls gegen ein unbeabsichtigtes Durchstoßen durch eine - nicht dargestellte - Schutzkappe oder andersartige sterile Abdeckung oder durch eine Kombination derselben geschützt.

Das Funktionsmodul 2 weist im Prinzip den gleichen Aufbau wie das Funktionsmodul 1 auf. Auch hier durchläuft eine Leitung 4 mit einem Einlass 5 und einem Auslass 6 das gesamte Funktionsmodul 2, und an zwei von außen gut zugänglichen und standardisierten Stellen sind zwei mit je einer Membran 15 verschlossene Portale 13, 14 vorgesehen. Im Unterschied zum Funktionsmodul 1 weist das Funktionsmodul 2 im Bereich des Auslasses 6 ein Priming-Ventil zum Entlüften des Funktionsmoduls 2 bzw. zum Befüllen des selben mit einem Fluid auf. Darüber hinaus unterscheidet sich das Funktionsmodul 2 vom Funktionsmodul 1 durch zwei Ventilstutzen 9, 10, welche am Einlass 5 und am Auslass 6 derart aus dem Gehäuse des Funktionsmoduls 2 herausragen, dass sie beim Aufsetzen des Funktionsmoduls 2 auf das Funktionsmodul 1 in die Portale 11, 12 des Funktionsmoduls 1 eingreifen. Dabei sind die Portale 11, 12 des Funktionsmoduls 1 derart ausgebildet, dass der Kreislauf der Körperflüssigkeit beim vollständigen Eingriff der Ventilstutzen 9, 10 in die Portale 11, 12 vom Modul 1 in das Modul 2 umgelenkt und somit der Funktionskreislauf des Moduls 1 überbrückt wird. Das gleiche kann durch Aufsetzen eines weiteren Funktionsmoduls 18 auf das Funktionsmodul 2 geschehen, wobei das Funktionsmodul 18 wiederum mit - hier nicht dargestellten - Ventilstutzen ausgestattet ist, die dann in die Portale 13, 14 des Funktionsmoduls 2 eingreifen.

Figur 2 zeigt eine Detaildarstellung eines ersten Ausführungsbeispiels der Portale 11, 12, 13, 14 in drei Phasen des Eingriffs eines Ventilstutzens 9. Diese erste Ausführungsform der Portale 11, 12, 13, 14 besteht darin, dass es eine winklige Abzweigung der zugehörigen Leitung 3, 4 enthält, wobei die Flussrichtung während des Betriebs des Funktionsmoduls 1 aus der Leitung 3, 4 rechtsherum in den abzweigenden Abschnitt 19 verläuft, da der Weg gerade aus in dieser Phase bzw. im normalen Arbeitszustand des Funktionsmoduls 1 noch durch eine Membran 15 verschlossen ist. In der Phase 2 (mittlere Darstellung) hat der Ventilstutzen 9 die Membran 15 durchstoßen und kommt somit in Eingriff mit dem Portal 11 des Funktionsmoduls 1. Zu diesem Zeitpunkt teilt sich der Körperflüssigkeitsstrom bereits auf in eine Richtung gerade aus in das Funktionsmodul 2 hinein und in eine verbleibende Teilströmung in den abzweigenden Abschnitt 19. Dieser Zustand besteht allerdings nur sehr kurze Zeit, nämlich gerade so lange, bis der Ventilstutzen 9 vollständig in das Portal 11 eingesteckt ist, was in der ganz rechten Darstellung gezeigt ist. Dann nämlich verschließt der Ventilstutzen 9 den abzweigenden Abschnitt 19 und der Durchfluss der Körperflüssigkeit wird in das zweite Funktionsmodul 2 vollständig umgeleitet.

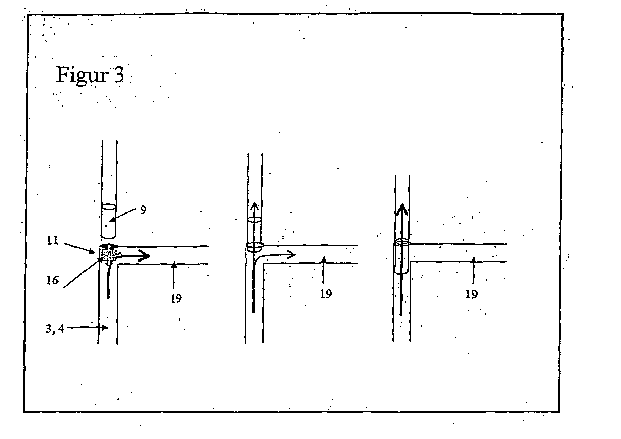

Figur 3 zeigt eine alternative Ausführungsform der Portale 11, 12, 13, 14, nach der in der Abzweigung ein Ventilmechanismus in Form eines Dreiwegeventils 16 angeordnet ist, mit welchem der Durchfluss der Körperflüssigkeit in das zweite Funktionsmodul 2 bzw. in jedes weitere Funktionsmodul 18 nach dem Eindringen des Ventilstutzens 9 umgeleitet wird. Bei dieser Ausführungsform braucht der Ventilstutzen 9 nicht so tief in das Portal 16 einzudringen, wie es bei der Ausführungsform gemäß Figur 2 der Fall ist, da der mit dem Eindringen einhergehende Verschluss des abzweigenden Abschnitts 19 bei der zweiten Ausführungsform gemäß Figur 3 mittels des Ventils 16 erfolgt.

## Patentansprüche

1. Vorrichtung zum Wechseln und/oder Andocken von Funktionsmodulen (1, 2) für die extrakorporale Zirkulation von Körperflüssigkeiten, wobei jedes Funktionsmodul (1, 2) wenigstens eine von der Körperflüssigkeit durchströmbare Leitung (3; 4) mit einem Einlass (5; 7) und einem Auslass (6; 8) aufweist,
**gekennzeichnet durch**
Mittel (9, 10; 11, 12) zur Verbindung der Leitung (3) eines ersten Funktionsmoduls (1) mit dem Einlass (5) und dem Auslass (6) eines zweiten Funktionsmoduls (2), derart, dass das erste Funktionsmodul (1) **durch** das zweite Funktionsmodul (2) strömungstechnisch überbrückt und die Körperflüssigkeit **durch** das zweite Funktionsmodul (2) umgeleitet wird.

2. Vorrichtung nach Anspruch 1,
**dadurch gekennzeichnet, dass**
die Verbindungsmittel (9, 10; 11, 12) je einen Ventilstutzen (9, 10) für den Einlass (5) und den Auslass (6) des zweiten Funktionsmoduls (2) sowie je ein zugeordnetes Portal (11, 12) in der Leitung (3) des ersten Funktionsmoduls (1) enthalten, wobei die Ventilstutzen (9, 10) beim Konnektieren der Funktionsmodule (1, 2) derart in das jeweils zugeordnete Portal (11, 12) eingreifen, dass der Funktionskreislauf des ersten Funktionsmoduls (1) überbrückt ist.

3. Vorrichtung nach Anspruch 2,
**dadurch gekennzeichnet, dass**
der Einlass (5) und der Auslass (6) des zweiten Funktionsmoduls (2) mit je einem Ventilstutzen (9, 10) versehen sind, die beim Zusammenstecken der Funktionsmodule (1, 2) mit den entsprechend ausgebildeten Portalen (11, 12) zur Herstellung einer flüssigkeitsdichten, von der Körperflüssigkeit durchströmbaren Verbindung zusammenwirken.

4. Vorrichtung nach Anspruch 2 oder 3,
**dadurch gekennzeichnet, dass**
das zweite Funktionsmodul (2) zum Anschluss eines weiteren Funktionsmoduls (18) ebenfalls mit Portalen (13, 14) versehen ist..

5. Vorrichtung nach Anspruch 2, 3 oder 4,
**dadurch gekennzeichnet, dass**
jedes Portal (11, 12; 13, 14) am nach außen weisenden Ende im ungebrauchten Zustand mittels einer durchstoßbaren Membran (15) keimfrei verschlossen ist.

6. Vorrichtung nach Anspruch 2, 3 oder 4,
**dadurch gekennzeichnet, dass**
jedes Portal (11, 12; 13, 14) am nach außen weisenden Ende mittels eines Ventils (16) keimfrei verschlossen ist.

7. Vorrichtung nach Anspruch 5 oder 6,
**dadurch gekennzeichnet, dass**
jedes Portal (11, 12; 13, 14) durch eine Schutzkappe oder Folie keimfrei oder steril gehalten wird.

8. Vorrichtung nach einem der vorstehenden Ansprüche,
**dadurch gekennzeichnet, dass**
der Auslass (6) des zweiten Funktionsmoduls (2) bzw. jedes weiteren Funktionsmoduls (18) mit einem Priming-Ventil (17) zum Entlüften des zweiten Funktionsmoduls (2) bzw. jedes weiteren Funktionsmoduls (18) oder zum Befüllen des selben mit einem Fluid versehen ist.

9. Vorrichtung nach einem der Ansprüche 2 - 8,
**dadurch gekennzeichnet, dass**
jedes Portal (11, 12, 13, 14) eine winklige Abzweigung der zugehörigen Leitung (3, 4) enthält, so dass jeder Ventilstutzen (9, 10) beim Eindringen in das ihm zugeordnete Portal (11, 12, 13, 14) den abzweigenden Abschnitt (19) der Leitung (3, 4) verschließt und den Durchfluss der Körperflüssigkeit in das zweite Funktionsmodul (2) bzw. in jedes weitere Funktionsmodul (18) umleitet.

10. Vorrichtung nach einem der Ansprüche 2 - 8,
**dadurch gekennzeichnet, dass**
jedes Portal einen Ventilmechanismus enthält, mit welchem der Durchfluss der Körperflüssigkeit in das zweite Funktionsmodul (2) bzw. in jedes weitere Funktionsmodul (18) nach dem Eindringen der Ventilstutzen (9, 10) umgeleitet wird.

## Claims

1. Device for changing and/or docking of functional modules (1, 2) for the extra corporeal circulation of body fluids wherein each functional module (1, 2) has at least one of the body fluid flow permeable conduits (3; 4) with an inlet (5; 7) and an outlet (6; 8),
**characterized by**
means (9, 10; 11, 12) for connecting the conduit (3) of the first functional module (1) to the inlet (5) and the outlet (6) of a second functional module (2) so that the first functional module (1) is fluidically bridged by the second functional module (2) and the body fluid is bypassed by the second functional module (2).

2. Device according to Claim 1,
**characterized in that**
the connecting means (9, 10; 11, 12) each contain a valve connection (9, 10) for the inlet (5) and the outlet (6) of the second functional module (2) as well as a designated portal (11, 12) in the conduit (3) of the first functional module (1) wherein the valve connections (9, 10) interact during the connecting of the functional modules (1, 2) in such a way in each of the assigned portals (11, 12) that the functional circulation of the first functional module (1) is by-passed.

3. Device according to Claim 2,
**characterized in that**
the inlet (5) and the outlet (6) of the second functional module (2), each having a valve connection (9, 10) which, upon connecting the functional module (1, 2) with the correspondingly designed portals (11, 12) for preparation of a leak-tight connection, where it can interact with the permeable body fluid.

4. Device according to Claim 2 or 3,
**characterized in that**
the second functional module (2) for connecting with an additional functional module (18) is also equipped with portals (13, 14).

5. Device according to one of the claims 2, 3, or 4,
**characterized in that**
each portal (11, 12; 13, 14) is sealed germfree at the outward pointing end in an unused condition by means of a permeable membrane (15).

6. Device according to one of the claims 2, 3, or 4,
**characterized in that**
each portal (11, 12; 13, 14) is sealed germfree at the outward pointing end by means of a valve (16).

7. Device according to one of the Claims 5 or 6
**characterized in that**
each portal (11, 12; 13, 14) is maintained germ free or sterile by means of a protective cap or foil.

8. Device according to one of the preceding claims
**characterized in that**
the outlet (6) of the second functional module (2) and/or of every additional functional module (18) is provided with a priming valve (17) for evacuation of the air from the second functional module (2) and/or from every additional functional module (18), or for filling the same with a fluid.

9. Device according to one of the Claims 2-8
**characterized in that**
each portal (11, 12, 13, 14) contains an angled branch of the associated conduit (3, 4) so that each connecting valve (9, 10) closes the axonal branch (19) of the conduit (3, 4) during a penetration into its assigned portal (11, 12, 13, 14) and diverts the flow of the body fluid into the second functional module (2) and/or in every other functional module (18).

10. Device according to one of Claims 2- 8
**characterized in that**
each portal contains a valve mechanism by means of which the flow of the body fluids is diverted into the second functional module (2) and/or into any additional functional module (18) after the penetration of the valve connections (9, 10).

## Revendications

1. Dispositif de remplacement et/ou de raccordement de modules fonctionnels (1, 2) destinés à la circulation extracorporelle de liquides corporels, dans lequel chaque module fonctionnel (1, 2) présente au moins un conduit (3; 4) qui est doté d'une entrée (5; 7) et d'une sortie (6; 8) et qui peut être traversé par le liquide corporel,
**caractérisé par**
des moyens (9, 10; 11, 12) de raccordement du conduit (3) d'un premier module fonctionnel (1) avec l'entrée (5) et la sortie (6) d'un deuxième module fonctionnel (2) de telle sorte que le premier module fonctionnel (1) soit contourné hydrauliquement par le deuxième module fonctionnel (2) et que le liquide corporel soit guidé dans le deuxième module fonctionnel (2).

2. Dispositif selon la revendication 1, **caractérisé en ce que** les moyens de raccordement (9, 10; 11, 12) contiennent chacun une tubulure de soupape (9, 10) respective pour l'entrée (5) et la sortie (6) du deuxième module fonctionnel (2) ainsi qu'une embouchure (11, 12) associée dans le conduit (3) du premier module fonctionnel (1), les tubulures de soupape (9, 10) s'engageant dans l'embouchure (11, 12) respective associée lors du raccordement des modules fonctionnels (1, 2), de telle sorte que le circuit fonctionnel du premier module fonctionnel (1) soit contourné.

3. Dispositif selon la revendication 2, **caractérisé en ce que** l'entrée (5) et la sortie (6) du deuxième module fonctionnel (2) sont chacune dotées d'une tubulure de soupape (9, 10) qui, lorsque les modules fonctionnels (1, 2) sont assemblés, coopère avec les embouchures (11, 12) de configuration appropriée pour établir un raccordement étanche aux liquides et qui peut être traversé par le liquide corporel.

4. Dispositif selon les revendications 2 ou 3, **caractérisé en ce que** le deuxième module fonctionnel (2) est également doté d'embouchures (13, 14) qui permettent le raccordement d'un autre module fonctionnel (18).

5. Dispositif selon les revendications 2, 3 ou 4, **caractérisé en ce que** lorsqu'elles ne sont pas utilisées, les embouchures (11, 12; 13, 14) sont fermées à leur extrémité tournée vers l'extérieur au moyen d'une membrane perforable (15) de manière à interdire l'accès aux germes.

6. Dispositif selon les revendications 2, 3 ou 4, **caractérisé en ce qu'**à son extrémité tournée vers l'extérieur, chaque embouchure (11, 12; 13, 14) est fermée au moyen d'une soupape de manière à en interdire l'accès aux germes.

7. Dispositif selon les revendications 5 ou 6, **caractérisé en ce que** chaque embouchure (11, 12; 13, 14) est maintenue sans germes ou stérile par un capuchon de protection ou un film.

8. Dispositif selon l'une des revendications précédentes, **caractérisé en ce que** la sortie (6) du deuxième module fonctionnel (2) ou de chaque autre module fonctionnel (18) est dotée d'une soupape d'amorçage (17) qui permet d'évacuer l'air du deuxième module fonctionnel (2) ou de chaque autre module fonctionnel (18) ou de les remplir d'un fluide.

9. Dispositif selon l'une des revendications 2 à 8, **caractérisé en ce que** chaque embouchure (11, 12; 13, 14) contient une dérivation coudée du conduit (3, 4) associé de telle sorte que lorsque chaque tubulure de soupape (9, 10) pénètre dans l'embouchure (11, 12; 13, 14) qui lui est associée, elle ferme la partie (19) dérivée du conduit (3, 4) et dévie le passage du liquide corporel vers le deuxième module fonctionnel (2) ou vers chaque autre module fonctionnel (18).

10. Dispositif selon l'une des revendications 2 à 8, **caractérisé en ce que** chaque embouchure contient un mécanisme de soupape par lequel le passage du liquide corporel vers le deuxième module fonctionnel (2) ou vers chaque autre module fonctionnel (18) est dévié après l'enfoncement de la tubulure de soupape (9, 10).
